# EUROPEAN PATENT SPECIFICATION

(11) **EP 2 819 596 B1**
(45) Date of publication and mention of the grant of the patent: **12.08.2015**
(21) Application number: 13723578.4
(22) Date of filing: 15.02.2013
(51) Int. Cl.: A61B 17/64, A61B 17/88, A61B 19/00

(54) **A FIXATION ROBOT FOR BONE FRACTURES**
FIXIERUNGSROBOTER FÜR KNOCHENBRÜCHE
ROBOT DE FIXATION POUR FRACTURES OSSEUSES

(30) Priority: 27.02.2012 TR 201202175
(43) Date of publication of application: 07.01.2015
(73) Proprietor: Tamis Otogaz Sanayi Ticaret Ve Muhendislik Hizmetleri Ithalat Ihracat Limited Sirketi, Samsun (TR)
(72) Inventor: KAHVECIOGLU, Hakan, Samsun (TR)
(74) Representative: Iskender, Ibrahim
(86) International application number: PCT/TR2013/000062
(87) International publication number: WO 2013/130023

(56) References cited:
- WO-A1-01/35842
- WO-A1-2010/138715
- US-A- 5 728 095

## Description

### Field of the Invention

The invention relates to a fixation robot, which performs the alignment of fractured bones automatically in the orthopedic operations carried out by means of an external fixation method and which is preferably controlled by means of a remote control.

### Prior Art

In the arm and leg fractures, when the physician decides to proceed with the external fixator treatment by taking the factors such as location and status of the fracture into account, the following known procedure is followed. Threaded nails are placed externally only on fractured bone fragments without making an incision over the fractured bones pieces Then, physician directs their assistants by looking at the screen of a so called fluoroscopy device (a camera showing live image by shooting X-ray video). Assistants acting on the order of the physician manually move the fractured bones pieces of the patient and try to align the fractured bones. However, in most operations, they cannot perform the reduction desired by the physician since they don't have enough power to overcome the resistance of the muscles around the fractured bone.

In the meantime, operating fluoroscopy device continuously and intensely emits X-ray (radiation). Although the patient receives this radiation once in their life, it is not a preferred situation for the physician to be exposed to this radiation. Thus, even though exact reduction desired is not attained, physician turns the fluoroscopy device off as soon as "enough" reduction is obtained. This situation increases the risk of never bonding or incorrect bonding of the fractured bones.

Even though the reduction is practised as the physician desired, assistants have to wait during one minute, which is the required time for the physician to secure the fixator (screwing the bolts), without changing their positions and the force they apply. In practice this is not quite possible as the staff become tired and reduces the force they apply unintentionally. Consequently, a poor or good reduction is provided, bolts of the fixator are tightened and the operation is terminated. External fixator is removed by the physician without the need for a second operation after bonding of the fractured bones at the end of the required time period and the treatment process is completed.

Patent no. WO 2002/069816 of Dutch origin is encountered during the patent search for the stabilization devices, so called fixator. The abstract of the patent application states the following: "The invention relates to an external fixation device for fractured bones having a carrying structure of interconnected elements comprising an extendible central body and clamps for bone screws respectively articulated on opposed ends of the central body."

The other patent application is patent no. WO 2001/091654 of Italian origin, belonging to ORTHOFIX S.R.L. In this application the following is stated: "The invention relates to a new type of axial unilateral external splint device for stabilizing bone fractures, comprising an extendible rod-like middle body (2) and oppositely located bone screw clamps (5, 6) which are articulated to respective ends (3, 4) of the rod-like middle body (2) by means of ball joints. Advantageously, a ball-and-socket joint (16) is mounted to each clamp (5, 6), within a main body (20) with which a bone screw clamping arrangement (25, 26, 21, 22, 23) is associated or co-operates."

The United States patent U.S. 5662648A discloses a fixation device having multi-axial movement and comprising a second mechanism in association with thereof through an intermediate member. Fixator secured on the bone by means of stabilizing elements (screws) is characterized in that it can be oriented with a second mechanism, however, in different axes, wherein a structure provided with freedom of movement by means of multi-axial movements is described.

Patent application no. WO2010/138715 A1 discloses the subject-matter of the preamble of claim 1.

### Object of the Invention

The objective of the present invention is to obtain a device having different technical specifications and bringing a new approach in this field as compared to the fixator configurations used in the current state of the art.

An objective of the present invention is to provide bringing bone ends into the same axis or aligning them automatically by means of a remote control without manual intervention and adjustment.

Another objective of the present invention is to protect physicians and other operating room personnel from the radiation emitted from fluoroscopy device during the operation by means of the remote control feature thereof.

Further objective of the present invention are to exactly align the two bone pieces in order to prevent possibility of not bonding of the fractured bone or minimize the delay in bonding; and to provide immobility of the bones during the time period required for securing the fixator.

Anther objective of the present invention is to realize a precise alignment thanks to the structure comprising a fixed jaw and a moving jaw, with the moving jaw having the ability to move with minimum parameters and to be just fixed with the desired parameter.

Another objective of the present invention is to provide high mobility thanks to the six-axis movement ability of the moving jaw.

A further objective of the present invention is to provide easy transportation of the fixation robot thanks to the portable structure as well as the advancing means thereof.

Another objective of the present invention is to minimize the treatment period of fractured bones and hence to carry out large number of treatments in shorter time periods. At the same time, the objective of the present invention is to minimize the effort spent by the physician and technical committee.

The present invention relates to a fixation robot as disclosed in appended independent claim 1. Preferred embodiments are disclosed in the dependent claims.

### Figures for a Better Understanding of the Invention

Figure 1 is a representational front view of the fixation robot according to the invention when fixator and bone are adapted.

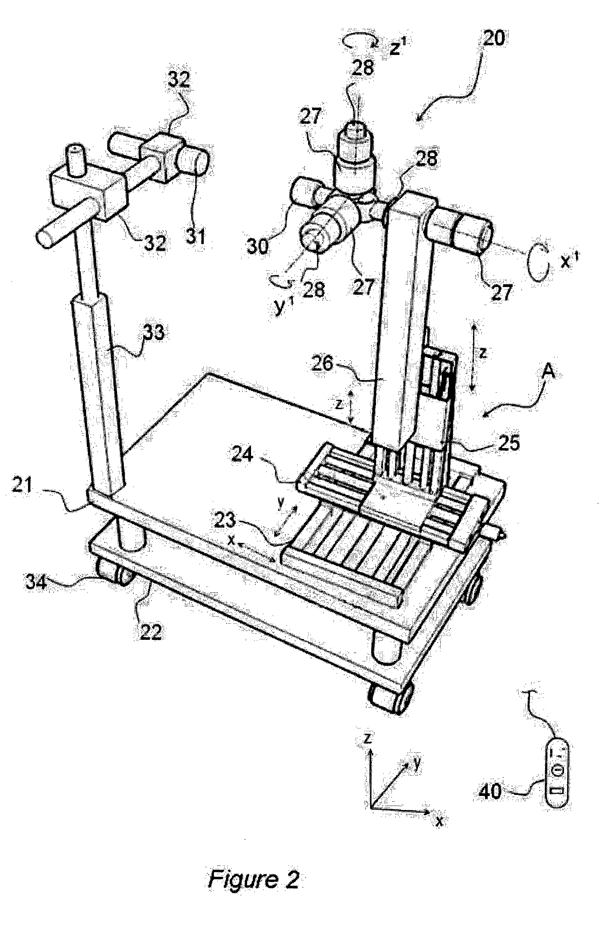

Figure 2 is an individual perspective view of the fixation robot according to the invention.
Figure 3 is a perspective view of the fixation robot according to the invention from a different angle.
Figure 4 is a close-up perspective view of the cardan shaft transferring motion to the moving jaw of the fixation robot according to the invention.

**Part Numbers**

| | |
|---|---|
| 10-Bone | 26-Moving column |
| 11-Connection nail | 27-Driving member |
| 12-Fixator | 28-Encoder |
| 20-Fixation robot | 29-Cardan shaft |
| 21-Carrying body | 30-Moving retainer jaw |
| 22-Lower plate | 31-Fixed retainer jaw |
| 23-Y-plane advancer | 32-Intermediate member |
| 24-X-plane advancer | 33-Fixed jaw arm |
| 25-Z-plane advancer | 34-Wheels |

### Detailed Description of the Invention

The present invention relates to a fixation robot (20) enabling the alignment of fractured bones (10) in orthopaedic operations, wherein it comprises the following: a fixed retainer jaw (31) connected to one piece of said fractured bone (10), a moving retainer jaw (30) connected to the other piece of said fractured bone (10) and a motion transfer assembly (A) providing motion to said moving retainer jaw (30).

Said motion transfer assembly (A) comprises a Y-plane advancer (23) parallel to the ground; an X-plane advancer (24) provided at an angle of 90° with respect to the Y-plane advancer (23) of the motion transfer assembly (A); a Z-plane advancer (25) moving vertically with respect to ground of the motion transfer assembly (A); a moving column (26) (A); a plurality of driving members (27) in association with said moving retainer jaw (30), wherein said driving members (27) are a motor and a reducer. Motion transfer assembly (A) also comprises encoders (28).

The fixation robot comprises a cardan shaft (29) associated with moving retainer jaw (30); nails (11) anchored on said bone (10); a fixator (12) located between these nails (11) and jaws (30, 31); an intermediate member (32) connected with said fixed retainer jaw (31); a fixed jaw arm (33); a carrying body (21) where said motion transfer assembly (A) is mounted; a lower plate (22) provided below said carrying body (21); wheels (34); and a hand controller (40) enabling the movement of said motion transfer assembly (A) in 6-axis.

### Operating method of the fixation robot (20)

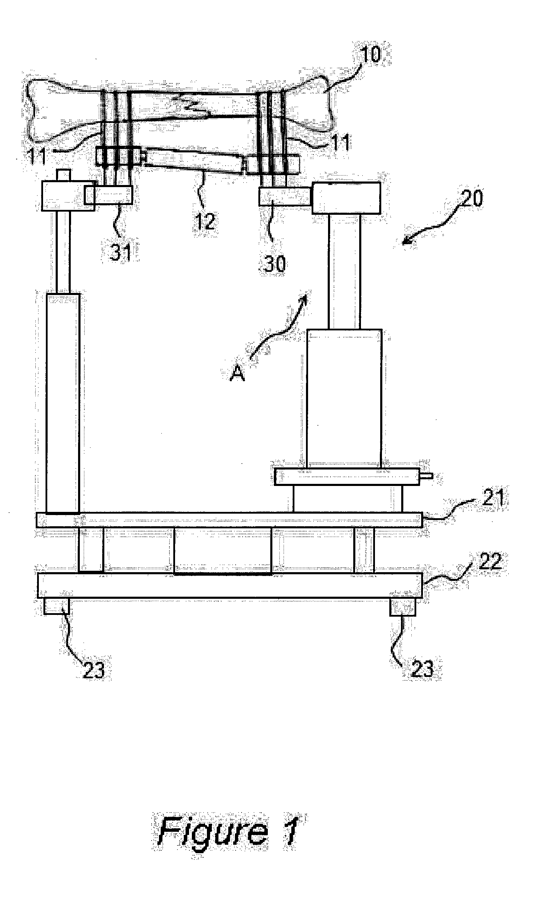

In figure 1, a representational two dimensional view of the fractured bone (10) held and fixed by the robot (20) is illustrated. One of the pieces of the two-piece fractured bone (10) is connected to the fixed retainer jaw (31) and the other one is connected to the moving retainer jaw (30) by means of nails (11) and/or different screw type connection members. Fixator (12) is preferably used between the bone (10) and the jaws (31, 30). The used fixator (12) is already an available fixator (12).

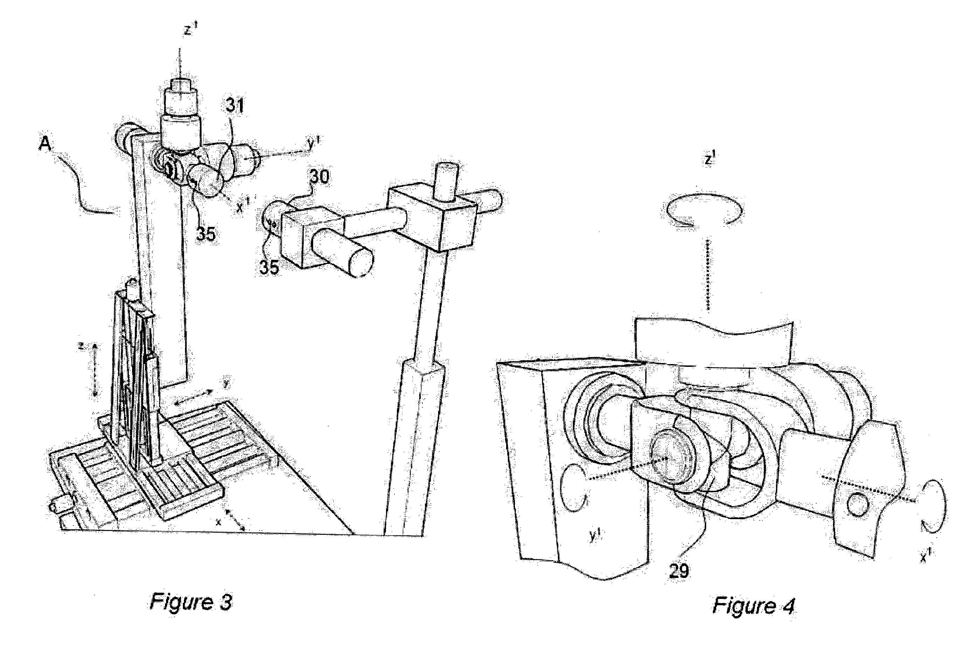

Moving retainer jaw (30) is provided on the carrying body (21) and it is capable of multi-axial movement. For example, the linear movement in y-plane is provided by the Y-plane advancer (23) while the movement in x-plane is provided by the X-plane advancer (24). The movement in the direction perpendicular to these planes (23, 24) is provided by the Z-plane advancer (25). Driving members (27) and cardan shaft (29) are provided on the Z-plane advancer (25) (see figure 4), enabling rotational movement of the moving jaw (30) in different axes.

Rotational movement of the moving retainer jaw (30) in the x¹-axis is provided by the driving member (27) comprising a motor and reducer assembly. Axial rotational movement in the y¹-axis and z¹-axis is also provided by the same driving members (27). Driving members (27) are associated with the encoder (28), wherein the parameters are controlled by this encoder (28). Cardan shaft (29) provides the rotational movement in three axes (x¹,y¹,z¹) by means of motors (see figure 4).

Fixed retainer jaw (31) is fixed on the carrying body (21) by means of intermediate members (32) and fixed jaw arm (33). Fixation robot (20) is provided with a portable construction and its transportation to any other location is possible by means of the wheels (34) thereof. This construction provides ease of use and practicality.

Nail slots (35) are formed on the moving and fixed retainer jaws (30, 31), wherein connection nails (11) providing the connection between the fixator (12) and bones (10) are located in said slots (35) (see figure 3). The fixator (12) used herein can be an intermediate component with a flexible structure as well as a mechanism having multiple joints.

## Claims

1. A fixation robot (20) enabling the alignment of fractured bones (10) in orthopaedic operations and comprising;
- a fixed retainer jaw (31) connected to one piece of said fractured bone (10);
- a moving retainer jaw (30) connected to the other piece of said fractured bone (10); and
- a motion transfer assembly (A) providing motion to said moving retainer jaw (30),
comprising a Y-plane advancer (23) parallel to the ground, and said motion transfer assembly (A) **characterized in that** it further comprises
- an X-plane advancer (24) at an angle of 90° with respect to the Y-plane advancer (23) and moving linearly on the Y-plane advancer (23),
- a Z-plane advancer (25) moving vertically with respect to the ground and moving linearly on the X-plane advancer (24),
- a moving column (26) carrying the moving retainer jaw (30) and moving linearly on the Z-plane advancer (25),
- a cardan shaft (29) associated with moving retainer jaw (30) and providing rotational movement to the moving retainer jaw (30) around the x, y and z axes by being driven plurality of motors and reducers from different directions.

2. A robot according to claim 1, wherein it comprises a motion transfer assembly (A) capable of moving in 6 axes comprising 3 degrees of translational movements and 3 degrees of rotational movements.

3. A robot according to any preceding claim, wherein said motion transfer assembly (A) comprises encoders (28).

4. A robot according to any preceding claim, wherein it comprises nails (11) anchored on said bone (10) and a fixator (12) provided between these nails (11) and jaws (30, 31).

5. A robot according to claim 1, wherein it comprises an intermediate member (32) connected with said fixed retainer jaw (31) and a fixed jaw arm (33).

6. A robot according to any preceding claim, wherein it comprises a carrying body (21) onto which said motion transfer assembly (A) is mounted.

7. A robot according to claim 6, wherein it comprises a lower plate (22) and wheels (34) located below the carrying body (21).

8. A robot according to any preceding claim, wherein it comprises a hand controller (40) enabling the movement of motion transfer assembly (A) in 6 axes comprising 3 degrees of translational movements and 3 degrees of rotational movements.

9. A robot according to any preceding claim, wherein it comprises nail slots (35) formed on said jaws (30, 31).

## Patentansprüche

1. Fixierungsroboter (20), der die Ausrichtung gebrochener Knochen (10) in orthopädischen Operationen ermöglicht und umfassend:
- eine feststehende Haltebacke (31), die mit einem Stück des gebrochenen Knochens (10) verbunden ist;
- eine bewegliche Haltebacke (30), die mit dem anderen Stück des gebrochenen Knochens (10) verbunden ist; und
- eine Bewegungsübertragungsanordnung (A), welche die Bewegung für die bewegbare Haltebacke (30) bereitstellt,
umfassend
einen Y-Ebenen-Vorschub (23) parallel zum Boden,
und die Bewegungsübertragungsanordnung (A) **dadurch gekennzeichnet ist, dass** sie ferner umfasst
- einen X-Ebenen-Vorschub (24) in einem Winkel von 90° in Bezug auf den Y-Ebenen-Vorschub (23) und sich geradlinig auf dem Y-Ebenen-Vorschub (23) bewegt,
- einen Z-Ebenen-Vorschub (25), der sich senkrecht in Bezug zum Boden bewegt und sich geradlinig auf dem X-Ebenen-Vorschub (24) bewegt,
- eine bewegliche Säule (26), welche die bewegbare Haltebacke (30) trägt und sich geradlinig auf dem Z-Ebenen-Vorschub (25) bewegt,
- eine Gelenkwelle (29), die mit der bewegbaren Haltebacke (30) verbunden ist und eine Drehbewegung für die bewegbare Haltebacke (30) rund um die x-, y- und z-Achsen bereitstellt, angetrieben durch eine Mehrzahl von Motoren und Reduzierstücken aus unterschiedlichen Richtungen.

2. Roboter nach Anspruch 1, wobei er eine Bewegungsübertragungsanordnung (A) aufweist, die zur Bewegung in 6 Achsen fähig ist, umfassend 3 Grade von Translationsbewegungen und 3 Grade von Drehbewegungen.

3. Roboter nach einem der vorhergehenden Ansprüche, wobei die Bewegungsübertragungseinheit (A) Encoder (28) aufweist.

4. Roboter nach einem der vorhergehenden Ansprüche, wobei er Nägel (11) aufweist, die an dem Knochen (10) verankert sind, und einer Fixiervorrichtung (12), der zwischen diesen Nägeln (11) und den Backen (30, 31) angeordnet ist.

5. Roboter nach Anspruch 1, wobei er ein Zwischenelement (32) aufweist, das mit der feststehenden Haltebacke (31) und einem feststehenden Backenarm (33) verbunden ist.

6. Roboter nach einem der vorhergehenden Ansprüche, wobei er einen Tragkörper (21) aufweist, auf dem die Bewegungsübertragungsanordnung (A) angebracht ist.

7. Roboter nach Anspruch 6, wobei er eine untere Platte (22) und Räder (34) aufweist, die sich unterhalb des Tragkörpers (21) befinden.

8. Roboter nach einem der vorhergehenden Ansprüche, wobei er eine Handsteuereinrichtung (40) aufweist, welche die Bewegung der Bewegungsübertragungsanordnung (A) in 6 Achsen ermöglicht, umfassend 3 Grade von Translationsbewegungen und 3 Grade von Drehbewegungen.

9. Roboter nach einem der vorhergehenden Ansprüche, wobei er Nagelaufnahmen (35) aufweist, die an den Backen (30, 31) ausgebildet sind.

## Revendications

1. Robot de fixation (20) permettant l'alignement d'os fracturés (10) au cours d'opérations orthopédiques et comprenant :
- une mâchoire de retenue fixe (31) reliée à une pièce de l'os fracturé (10) ;
- une mâchoire de retenue mobile (30) reliée à l'autre pièce de l'os fracturé (10) ; et
- un assemblage de transfert de mouvement (A) fournissant un mouvement à la mâchoire de retenue mobile (30)
comprenant
un système d'avancée en plan Y (23) parallèle au sol,
et l'assemblage de transfert de mouvement (A) étant **caractérisé en ce qu'**il comprend en outre
- un système d'avancée en plan X (24) à un angle de 90° par rapport au système d'avancée en plan Y (23) et se déplaçant de façon linéaire sur le système d'avancée en plan Y (23),
- un système d'avancée en plan Z (25) se déplaçant verticalement par rapport au sol et se déplaçant de façon linéaire sur le système d'avancée en plan X (24),
- une colonne mobile (26) supportant la mâchoire de retenue mobile (30) et se déplaçant de façon linéaire sur le système d'avancée en plan Z (25),
- un arbre à cardan (29) associé à la mâchoire de retenue mobile (30) et fournissant un mouvement de rotation à la mâchoire de retenue mobile (30) autour des axes x, y et z en étant entraîné par une pluralité de moteurs et de réducteurs de différentes directions.

2. Robot selon la revendication 1, comprenant un assemblage de transfert de mouvement (A) capable de se déplacer dans 6 axes comprenant 3 degrés de mouvements de translation et 3 degrés de mouvements de rotation.

3. Robot selon l'une quelconque des revendications précédentes, dans lequel l'assemblage de transfert de mouvement (A) comprend des encodeurs (28).

4. Robot selon l'une quelconque des revendications précédentes, comprenant des clous (11) ancrés sur l'os (10) et un fixateur (12) prévu entre ces clous (11) et les mâchoires (30, 31).

5. Robot selon la revendication 1, comprenant un élément intermédiaire (32) relié à la mâchoire de retenue fixe (31) et un bras de mâchoire fixe (33).

6. Robot selon l'une quelconque des revendications précédentes, comprenant un corps de transport (21) sur lequel l'assemblage de transfert de mouvement (A) est monté.

7. Robot selon la revendication 6, comprenant une plaque inférieure (22) et des roues (34) situées sous le corps de transport (21).

8. Robot selon l'une quelconque des revendications précédentes, comprenant une commande manuelle (40) permettant le mouvement de l'assemblage de transfert de mouvement (A) dans 6 axes comprenant 3 degrés de mouvements de translation et 3 degrés de mouvements de rotation.

9. Robot selon l'une quelconque des revendications précédentes, comprenant des fentes à clou (35) formées sur les mâchoires (30, 31).
